# EUROPEAN PATENT APPLICATION

(11) **EP 4 548 877 A2**
(43) Date of publication of application: **07.05.2025**
(21) Application number: 25164471.2
(22) Date of filing: 15.12.2017
(51) Int. Cl.: A61F 2/16

(54) **INTRAOCULAR LENS INJECTOR**

(30) Priority: 19.12.2016 JP 2016245504; 19.12.2016 JP 2016245505
(62) Divisional of application: 17884933.7
(71) Applicant: Santen Pharmaceutical Co., Ltd., Osaka-shi, Osaka 533-8651 (JP)
(72) Inventor: MATSUMOTO, Kazuma, Osaka, 533-8651 (JP)
(74) Representative: Müller-Boré & Partner Patentanwälte PartG mbB

(57) **Abstract**

An intraocular lens injector comprising: a lens holder configured to hold an intraocular lens which has a lens part and a first support part and a second support part which curvedly extend point-symmetrically with respect to the lens part; a plunger configured to extrude the intraocular lens from a rear side of the intraocular lens injector; and a nozzle through which to externally release the intraocular lens, which has been extruded by the plunger, while folding the intraocular lens, the lens holder holding the intraocular lens so that the first support part extends rearward and the second support part extends forward, said intraocular lens injector further comprising: a meandering mechanism which, assuming that with reference to a central line passing through a center of the lens part and extending in a forward-rearward direction, a side of the lens part which side is closer to a root of the first support part is regarded as a first side and a side of the lens part which side is opposite from the first side is regarded as a second side, has a contact part which is provided at an entrance of the nozzle and comes into contact with a tip of the plunger, the meandering mechanism causing a course of the plunger to meander to the second side by a contact between the contact part and the plunger.

## Description

### Technical Field

The present invention relates to an injector for an intraocular lens (hereinafter referred to as an "intraocular lens injector").

### Background Art

An intraocular lens injector which is configured to release an intraocular lens, held by a lens holder, by causing a plunger to extrude the intraocular lens is known as a conventional technique (e.g., Patent Literature 1). The intraocular lens injector described in Patent Literature 1 includes a nozzle through which to release, by an operation to push the plunger, the intraocular lens, held by the lens holder, while folding the intraocular lens in a recessed shape. The nozzle is configured to have an inner diameter which is gradually made smaller as the nozzle extends forward.

Furthermore, a lens holder provided to an intraocular lens injector described in Patent Literature 2 has (i) a placement part in which to place an intraocular lens and (ii) a lid part which is openably connected with the placement part. The intraocular lens is placed in the placement part, and then the lid part is closed, so that the intraocular lens is held by the lens holder.

### Citation List

### [Patent Literatures]

[Patent Literature 1]
   Japanese Patent Application Publication, Tokukai, No. 2014-140711
[Patent Literature 2]
   Japanese Patent Application Publication, Tokukai, No. 2014-100558

### Summary of Invention

### Technical Problem

According to the technique described in Patent Literature 1, the operation to push the plunger may cause a support part of the intraocular lens which support part is in contact with a tapered surface of the nozzle to be bent in a rearward unintended direction depending on a location at which the intraocular lens and a tip of the plunger are in contact with each other. Thus, a conventional intraocular lens injector has room for improvement in terms of restraint of bending of an intraocular lens.

According to the technique described in Patent Literature 2, a plunger which should extrude the intraocular lens, held by the lens holder, by coming into contact with the intraocular lens may override the intraocular lens or may pass under the intraocular lens. This causes a problem of difficulty in suitably extruding an intraocular lens.

An aspect of the present invention has a first object to achieve an intraocular lens injector which is capable of releasing an intraocular lens while restraining the intraocular lens from being bent. Furthermore, an aspect of the present invention has a second object to achieve an intraocular lens injector which is capable of suitably extruding an intraocular lens.

### Solution to Problem

In order to attain the objects, an intraocular lens injector in accordance with an aspect of the present invention includes: a lens holder configured to hold an intraocular lens which has a lens part and a first support part and a second support part which curvedly extend point-symmetrically with respect to the lens part; a plunger configured to extrude the intraocular lens from a rear side of the intraocular lens injector; and a nozzle through which to externally release the intraocular lens, which has been extruded by the plunger, while folding the intraocular lens, the lens holder holding the intraocular lens so that the first support part extends rearward and the second support part extends forward, the intraocular lens injector further including: a meandering mechanism which, assuming that with reference to a central line passing through a center of the lens part and extending in a forward-rearward direction, a side of the lens part which side is closer to a root of the first support part is regarded as a first side and a side of the lens part which side is opposite from the first side is regarded as a second side, has a contact part which is provided at an entrance of the nozzle and comes into contact with a tip of the plunger, the meandering mechanism causing a course of the plunger to meander to the second side by a contact between the contact part and the plunger.

In order to attain the objects, a further intraocular lens injector in accordance with an aspect of the present invention includes: a body which is tubular; a lens holder provided on an end surface of the body and configured to hold an intraocular lens; a plunger to be inserted into the body, while being capable of entering and leaving the body, so as to (i) come into contact with the intraocular lens which is held by the lens holder and (ii) extrude the intraocular lens; and a nozzle through which to release the intraocular lens, which has been extruded by the plunger, while folding the intraocular lens, the lens holder having: a placement part which is provided with a placement surface on which to place the intraocular lens; and a lid part which is provided so as to cover the placement part, the lid part being provided with a pressing member configured to press the intraocular lens toward the placement surface by an elastic force.

In order to attain the objects, a further intraocular lens injector in accordance with an aspect of the present invention includes: a body which is tubular; a lens holder provided on an end surface of the body and configured to hold an intraocular lens; a plunger to be inserted into the body, while being capable of entering and leaving the body, so as to (i) come into contact with the intraocular lens which is held by the lens holder and (ii) extrude the intraocular lens; and a nozzle through which to release the intraocular lens, which has been extruded by the plunger, while folding the intraocular lens, the lens holder having: a placement part which is provided with a placement surface on which to place the intraocular lens; and a lid part which is provided so as to cover the placement part and configured to press the intraocular lens toward the placement surface by an elastic force.

### Advantageous Effects of Invention

An aspect of the present invention brings about an effect of releasing an intraocular lens while restraining the intraocular lens from being bent. Furthermore, an aspect of the present invention brings about an effect of achieving an intraocular lens injector which is capable of suitably extruding an intraocular lens.

### Brief Description of Drawings

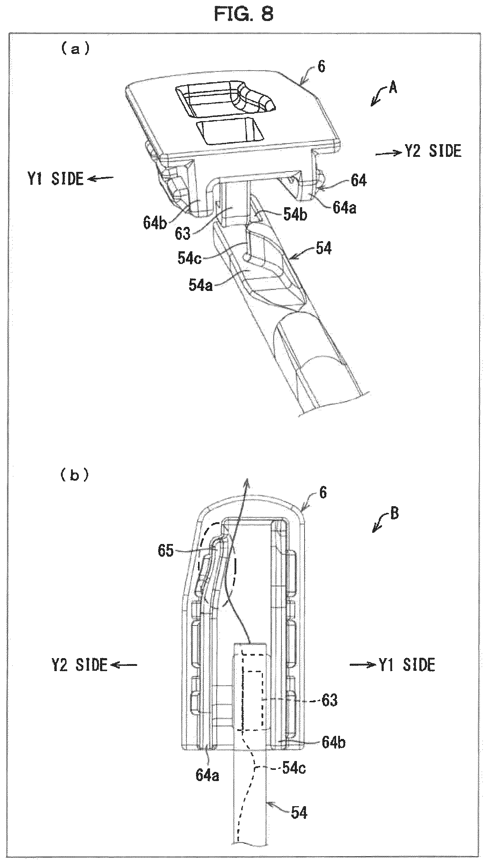

Fig. 1 is a perspective view illustrating an overall configuration of an intraocular lens injector in accordance with Embodiment 1 of the present invention.
Fig. 2 is a perspective view illustrating a configuration of a device body of the intraocular lens injector in accordance with Embodiment 1 of the present invention.
Fig. 3 is a perspective view illustrating a configuration of a tip chip of the intraocular lens injector in accordance with Embodiment 1 of the present invention.
Fig. 4 illustrates a configuration of a lens holder of the intraocular lens injector in accordance with Embodiment 1 of the present invention. (a) of Fig. 4 is a perspective view of the lens holder, and (b) of Fig. 4 is a bottom view of the lens holder which is viewed from its lower side.
Fig. 5 is a perspective view illustrating a configuration of a plunger of an intraocular lens injector in accordance with an embodiment of the present invention.
Fig. 6 illustrates a configuration of a plunger guide of the intraocular lens injector in accordance with Embodiment 1 of the present invention. (a) of Fig. 6 is a perspective view of the plunger guide, and (b) of Fig. 6 is a bottom view of the plunger guide which is viewed from its lower side.
Fig. 7 is a perspective view illustrating how a plunger and an intraocular lens are provided in a case where the intraocular lens is set in a lens holder.
   (a) of Fig. 8 is a perspective view illustrating a configuration of a meandering mechanism, which is a characteristic feature of the intraocular lens injector in accordance with Embodiment 1 of the present invention. (b) of Fig. 8 illustrates a configuration of a course changing mechanism of an intraocular lens injector in accordance with an embodiment of the present invention and is a view of a plunger guide which is viewed from its lower side.

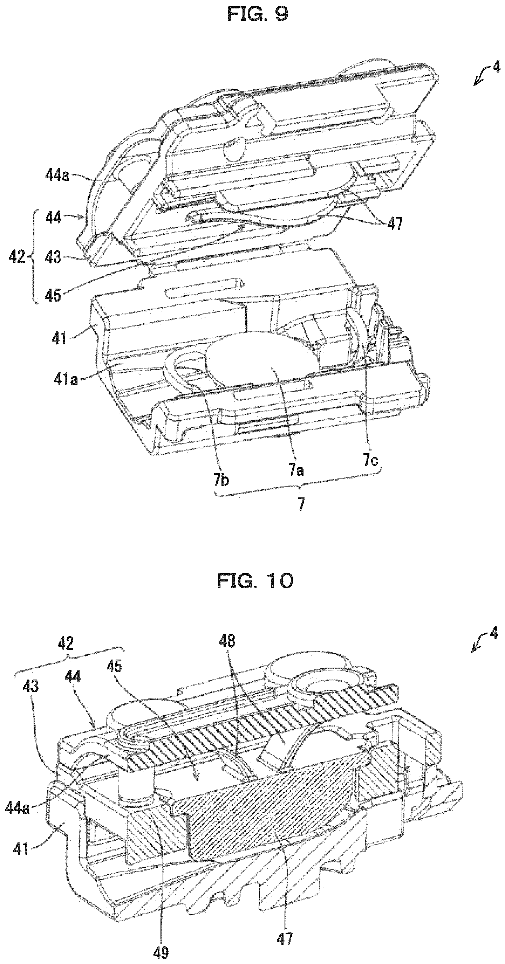

Fig. 9 is a perspective view illustrating a state in which an intraocular lens is placed in a bottom part of a lens holder of an intraocular lens injector in accordance with Embodiment 2 of the present invention and a lid part of the lens holder is open.
Fig. 10 is a perspective, cross-sectional view illustrating a configuration of the bottom part and the lid part which are provided in the lens holder.

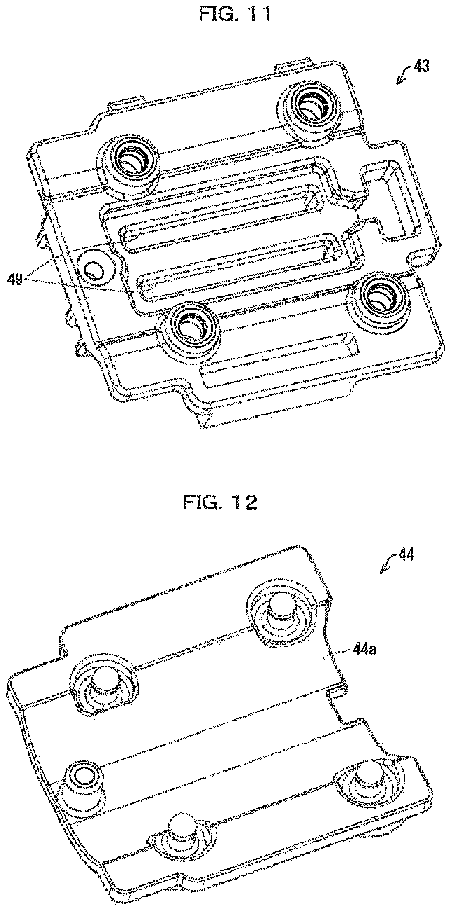

Fig. 11 is a perspective view illustrating a groove member which is provided in the lid part.
Fig. 12 is a perspective view illustrating an overcover member which is provided in the lid part.

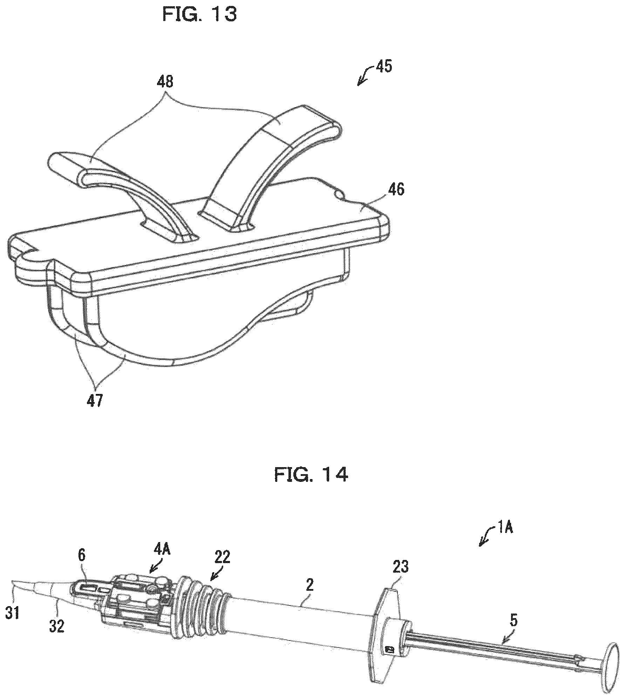

Fig. 13 is a perspective view illustrating a fitted rib member which is provided in the lid part.
Fig. 14 is a perspective view illustrating an overall configuration of an intraocular lens injector in accordance with Embodiment 3 of the present invention.

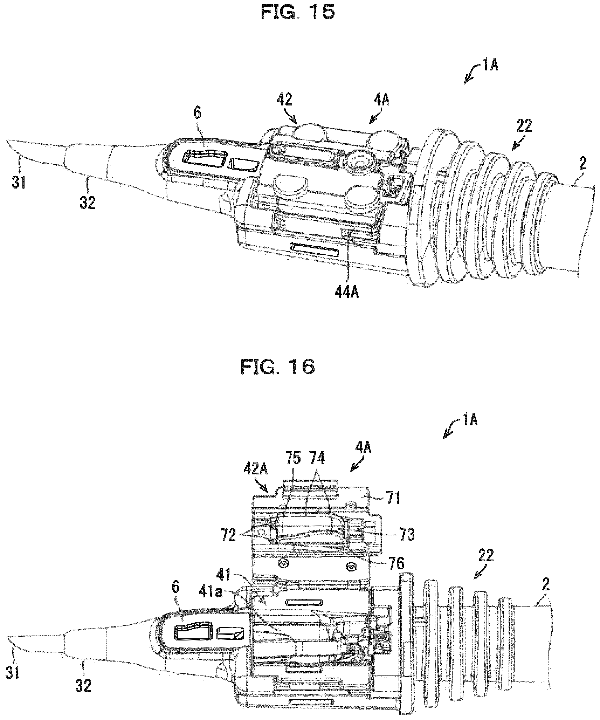

Fig. 15 is an enlarged perspective view illustrating a part of the intraocular lens injector which part is provided with a lens holder.
Fig. 16 is an enlarged perspective view illustrating a state in which a lid part which is provided in the lens holder is opened.

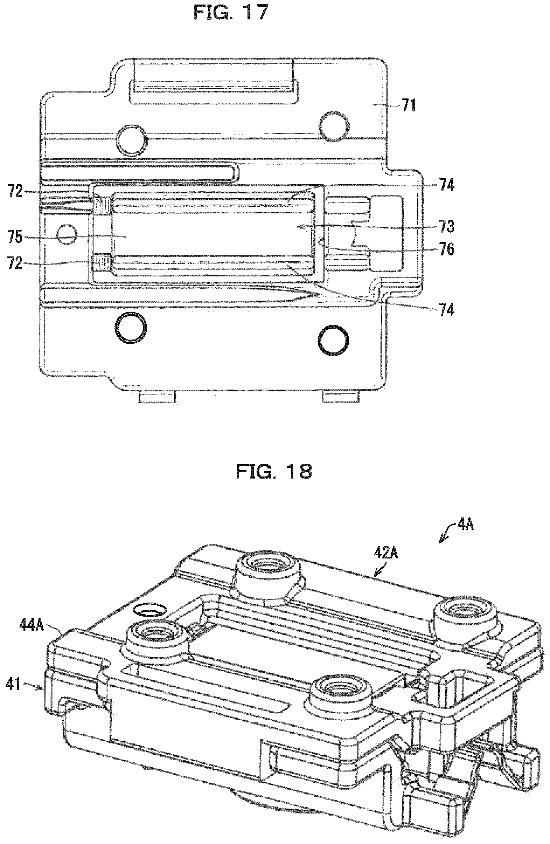

Fig. 17 is a bottom view of the lid part.
Fig. 18 is a perspective view illustrating the lens holder which is in a closed state.

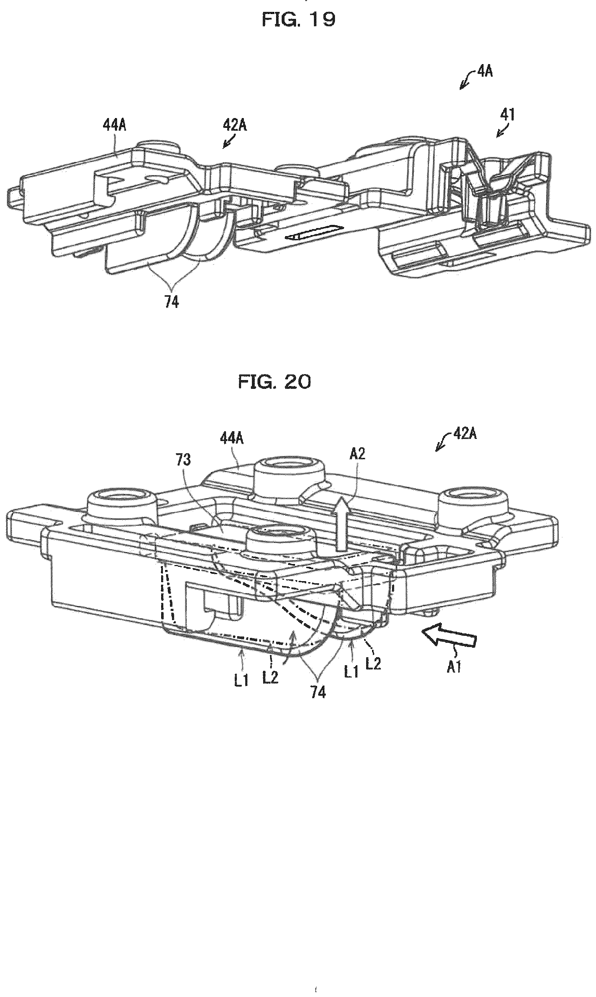

Fig. 19 is a perspective view illustrating the lens holder which is in an opened state.
Fig. 20 is a perspective view for describing an operation carried out by the lens holder.

### Description of Embodiments

### [Embodiment 1]

The following description will specifically discuss an embodiment of the present invention.

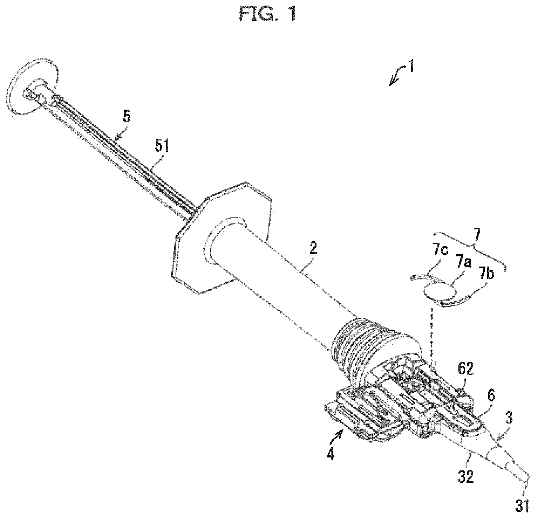

Fig. 1 a perspective view illustrating an overall configuration of an intraocular lens injector 1 (hereinafter referred to as an "injector 1"). As illustrated in Fig. 1, the injector 1 includes a device body 2 which is tubular, a tip chip 3, a lens holder 4 configured to hold an intraocular lens 7, a plunger 5, and a plunger guide 6. The tip chip 3 has a nozzle 32 which is connected to the device body 2. The lens holder 4 is attachable to the tip chip 3. The plunger 5 is rod-shaped and is configured to be inserted into the device body 2. The plunger guide 6 is provided in a part, behind the nozzle 32, of the tip chip 3.

Note that a direction in which a core of a shaft of the plunger 5 extends is herein regarded as a forward-rearward direction. Furthermore, a direction in which a thickness of the intraocular lens 7 which is held by the lens holder 4 extends is regarded as an upward-downward direction, and a direction which is perpendicular to both the forward-rearward direction and the upward-downward direction is regarded as a rightward-leftward direction. Moreover, a side of the injector 1 on which side the tip chip 3 is provided is regarded as a front side, and a side of the injector 1 which side is opposite from the front side is regarded as a rear side. Further, a side of the tip chip 3 on which side the plunger guide 6 is provided is regarded as an upper side, and a side of the tip chip 3 which side is opposite from the upper side is regarded as a lower side.

Note here that the intraocular lens 7 includes a lens part 7a, a front support part 7b, and a rear support part 7c. The lens part 7a is a lens part which functions as a crystalline lens after the intraocular lens 7 has been inserted in an eye. The front support part 7b and the rear support part 7c are each formed so as to curvedly extend from a side surface of the lens part 7a and each have a function of supporting, in an eye, the lens part 7a which has been inserted in the eye. The front support part 7b and the rear support part 7c are provided in a positional relationship in which the front support part 7b and the rear support part 7c are symmetric with respect to a point, which is a center of the lens part 7a. The lens part 7a, the front support part 7b, and the rear support part 7c are each made of an elastic material and deformably formed. The description of Embodiment 1 takes, as an example, the intraocular lens 7 of a single piece type which intraocular lens 7 is obtained by integrally forming the lens part 7a, the front support part 7b, and the rear support part 7c.

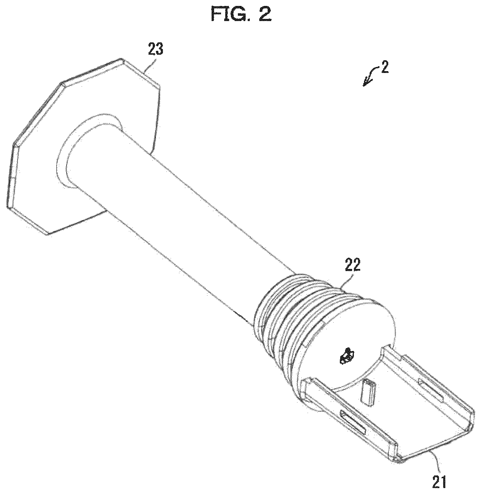

Fig. 2 is a perspective view illustrating a configuration of the device body 2. As illustrated in Fig. 2, the device body 2 (i) has, at its front, a bottom part 21 which is engaged with the tip chip 3, (ii) has, on its front end outer surface, a plurality of circular protruding parts 22 which can be held by an operator, and (iii) has, on its rear end outer surface, a holding part 23 which is flange-shaped and is held by an operator while a finger of the operator is caught in the holding part 23. The device body 2 is made of a shock-resistant resin such as a polycarbonate.

Note that the plurality of circular protruding parts 22 and the holding part 23 each can have any shape that allows a needed function to be carried out. For example, the holding part 23 does not necessarily need to be flange-shaped but can be constituted by, for example, a protrusion in which a finger can be caught.

By pushing the plunger 5 into the device body 2 with use of one of his/her hands, and, concurrently, holding the circular protruding parts 22 with use of the other of those hands, an operator releases the intraocular lens 7 into an eye of a patient so that the front support part 7b, the lens part 7a, and the rear support part 7c are released in this order. The device body 2 which is provided with the circular protruding parts 22 makes it easier for an operator to hold the injector 1, and consequently allows the injector 1 to be more operable.

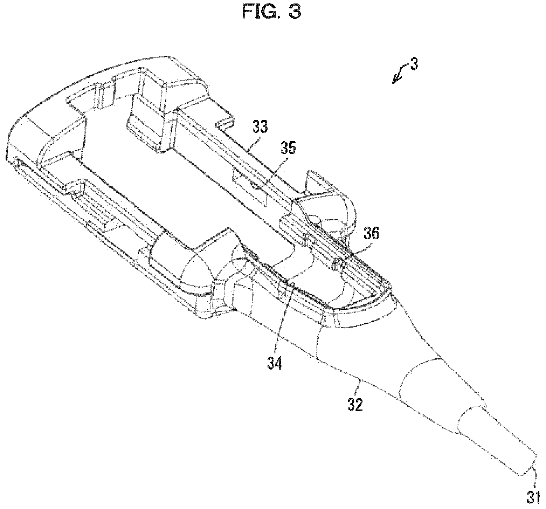

Fig. 3 is a perspective view illustrating a configuration of the tip chip 3. As illustrated in Fig. 3, the tip chip 3 has (i) a release part 31 from which to release the intraocular lens 7, (ii) the nozzle 32 whose inner diameter is gradually made smaller as the nozzle 32 extends forward, (iii) a rectangular part 33 whose center is opened and has a rectangular outer circumference, and (iv) an opening 34 in which to provide the plunger guide 6. The tip chip 3 is connected to the device body 2 by engaging the rectangular part 33 with the bottom part 21. Note that the tip chip 3 and the device body 2 can be engaged with each other by any method, e.g., with use of a locking claw and a locking hole. Note also that the tip chip 3 is made of a chemical-resistant and flexible resin such as a polypropylene or a polyamide.

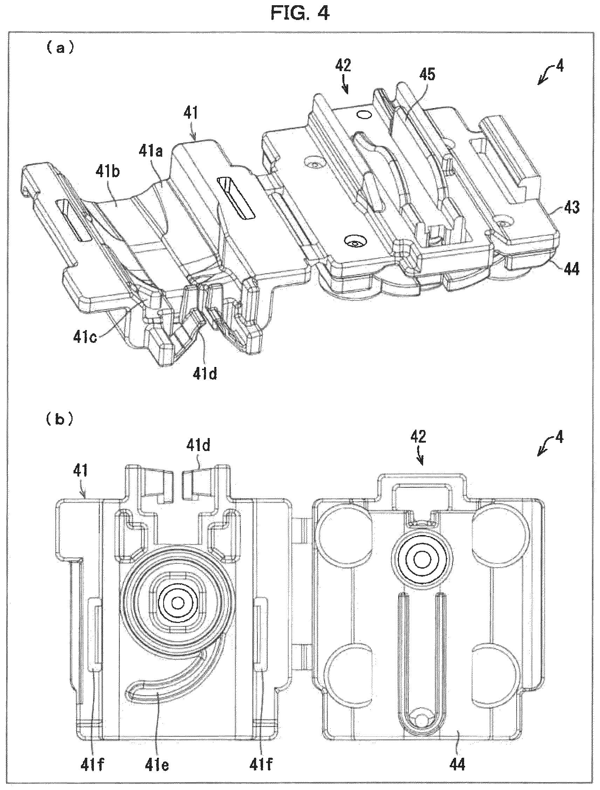

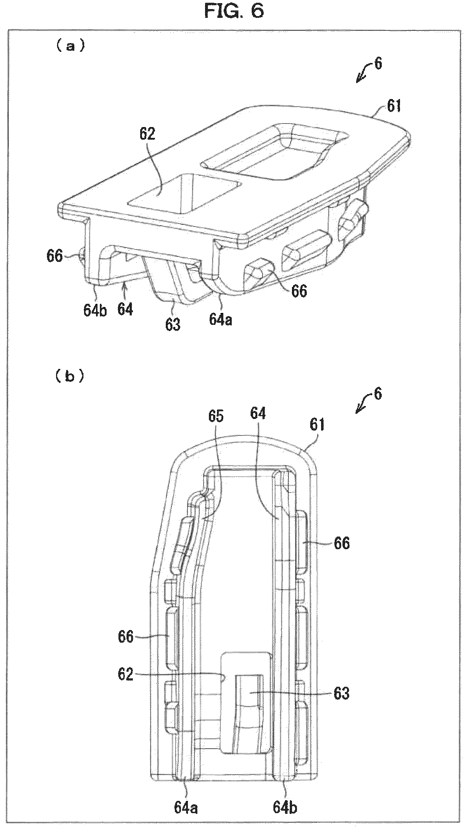

The rectangular part 33 has an engagement hole 35 which is to be engaged with a locking claw 41f (Fig. 4) of the lens holder 4. The opening 34 is provided in front of the rectangular part 33. Furthermore, the opening 34 has, on its side wall which extends in the forward-rearward direction, an engagement recess 36 which is to be engaged with an engagement protrusion 66 (Fig. 6) of the plunger guide 6.

The lens holder 4 can be inserted in an opening of the rectangular part 33 in advance of use of the injector 1. Alternatively, the lens holder 4 can be inserted in the opening of the rectangular part 33 during use of the injector 1.

Fig. 4 illustrates a configuration of the lens holder 4. (a) of Fig. 4 is a perspective view of the lens holder 4, and (b) of Fig. 4 is a bottom view of the lens holder 4 which is viewed from its lower side. As illustrated in (a) of Fig. 4, the lens holder 4 is constituted by a bottom part 41 and a lid part 42 which is openably connected with the bottom part 41. The intraocular lens 7 is held by placing the intraocular lens 7 in the bottom part 41 and then closing the lid part 42. The lens holder 4 is made of a chemical-resistant resin such as a polypropylene.

The bottom part 41 of the lens holder 4 has a placement surface 41a having a central part in which to place the intraocular lens 7. The placement surface 41a has, at its center, a groove part 41b which extends in the forward-rearward direction. The bottom part 41 has a lens position regulating part 41c and a plunger position regulating part 41d. The lens position regulating part 41c (i) has a function of regulating a rear end position of the intraocular lens 7 which has been placed on the placement surface 41a and (ii) has a bent shape which conforms with a shape of the rear support part 7c. The plunger position regulating part 41d is provided at a rear end of the bottom part 41 and has a holding part configured to hold a tip of the plunger 5. The plunger position regulating part 41d regulates a position of the plunger 5 relative to the intraocular lens 7.

Furthermore, as illustrated in (b) of Fig. 4, the bottom part 41 has a lower surface which is provided with a placement guiding part 41e which is shaped in a form of the intraocular lens 7. In a case where the bottom part 41 is seen from above, the placement guiding part 41e is visible through the placement surface 41a and thus is recognizable to an operator. Thus, in a case where an operator places the intraocular lens 7 in accordance with the placement guiding part 41e which is visible through the placement surface 41a, the intraocular lens 7 is provided at a suitable position in the placement surface 41a of the bottom part 41.

Furthermore, the bottom part 41 has an outer surface which is provided with the locking claw 41f which is to be engaged with the engagement hole 35 (Fig. 3) of the tip chip 3. Note that it is possible to engage the locking claw 41f of the lens holder 4 with the bottom part 21 of the device body 2 without providing the tip chip 3 with the engagement hole 35.

The lid part 42 includes a groove member 43, an overcover member 44, and a fitted rib member 45. In a case where the lid part 42 is closed, the groove member 43 (i) is provided so as to be opposite to the placement surface 41a with respect to the intraocular lens 7 and (ii) is provided with a pair of hollows which faces the intraocular lens 7. The overcover member 44 is (a) provided so as to be opposite to the placement surface 41a with respect to the groove member 43 and (b) provided so as to cover the hollows of the groove member 43. The fitted rib member 45 has a function of pressing the intraocular lens 7 toward the placement surface 41a by an elastic force by passing through the hollows of the groove member 43 and coming into contact with the overcover member 44 and the intraocular lens 7 which has been placed on the placement surface 41a.

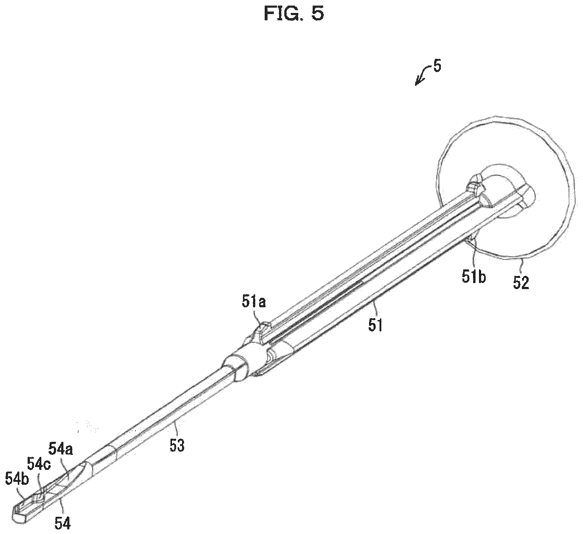

Fig. 5 is a perspective view illustrating a configuration of the plunger 5. As illustrated in Fig. 5, the plunger 5 includes a rear side shaft 51, a pressing part 52, a middle shaft 53, and an L-shaped part 54.

The rear side shaft 51 is located on the rearmost side of the plunger 5. The rear side shaft 51 is a part which is exposed to an outside of the device body 2 in a state in which the plunger 5 is located at its initial position at which the plunger 5 is inserted in the device body 2 (see Fig. 1). Furthermore, the rear side shaft 51 has a locking part 51a which is locked at a rear end of the device body 2, and has, on its rear side, a stopper 51b which protrudes outward. The pressing part 52 is provided on a rear-side outer circumferential surface of the rear side shaft 51 so as to be flange-shaped. An operator pushes the pressing part 52 toward the device body 2 so as to move the plunger 5 to a tip side of the injector 1.

The middle shaft 53 is connected to a front side of the rear side shaft 51 and is a shaft which is smaller in diameter than the rear side shaft 51. The middle shaft 53 has, at is tip, the L-shaped part 54 whose cross section is L-shaped.

The L-shaped part 54 is constituted by a bottom part 54a and a side wall part 54b. The bottom part 54a is shaped so as to have a smaller thickness in the upward-downward direction as the bottom part 54a extends toward the tip of the plunger 5. The side wall part 54b is provided at an end located on one of the right side and the left side of the bottom part 54a. Furthermore, the L-shaped part 54 is provided with a projecting part 54c which projects toward an end located on the other, on which no side wall part 54b is provided, of the right side and the left side of the bottom part 54a.

Moreover, an elastic member (not illustrated) such as a spring can be provided between an outer circumferential surface of the middle shaft 53 and an inner circumferential surface of the device body 2 so as to be parallel with the outer circumferential surface and the inner circumferential surface. With the configuration, the plunger 5 which is being pushed into the device body 2 receives a reaction force from the elastic member, so that the intraocular lens 7 can be prevented from bursting out of the L-shaped part 54 of the plunger 5. The elastic member can be located at any position that is not particularly limited, e.g., between the pressing part 52 of the plunger 5 and a rear end of the device body 2, provided that the plunger 5 can receive a reaction force from the elastic member while being pushed into the device body 2.

Fig. 6 illustrates a configuration of the plunger guide 6. (a) of Fig. 6 is a perspective view of the plunger guide 6, and (b) of Fig. 6 is a bottom view of the plunger guide 6 which is viewed from its lower side. The plunger guide 6 is a member which guides, to the release part 31 of the tip chip 3, the tip (L-shaped part 54) of the plunger 5 which has been pushed forward by an operation, carried out by an operator, to push the plunger 5 and then has passed through the lens holder 4. The plunger guide 6 is provided in the opening 34 of the tip chip 3. As illustrated in (a) of Fig. 6, the plunger guide 6 has (i) a blocking part 61 which is plate-shaped and (ii) a passage part 64 which is provided below the blocking part 61. The blocking part 61 (i) has a size which is substantially identical to a size of the opening 34 of the tip chip 3 and (ii) is a part which blocks the opening 34. The passage part 64 is a passage through which to guide the L-shaped part 54 of the plunger 5 to the release part 31 and has two wall parts 64a and 64b which face each other and extend in the forward-rearward direction.

Furthermore, the passage part 64 has an outer surface which is provided with the engagement protrusion 66. The engagement protrusion 66 is engaged with the engagement recess 36 which is provided on the side wall of the opening 34 of the tip chip 3.

Moreover, the blocking part 61 is provided with a downward protruding part 63 which protrudes downward. The downward protruding part 63 is provided between the two wall parts 64a and 64b which constitute the passage part 64. The downward protruding part 63 is provided so as to slide in the bottom part 54a and the side wall part 54b of the L-shaped part 54 in a case where the plunger 5 enters the passage part 64 (Fig. 8). The downward protruding part 63 has a function of keeping a course, on which the plunger 5 moves toward the release part 31, constant by preventing a shift of the course by coming into contact with the L-shaped part 54 of the plunger 5 so as to slide in the bottom part 54a and the side wall part 54b.

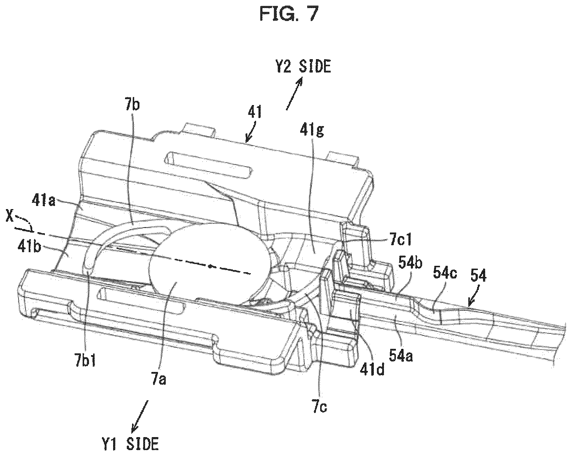

Fig. 7 is a perspective view illustrating how the plunger 5 and the intraocular lens 7 are provided in a case where the intraocular lens 7 is set in the bottom part 41 of the lens holder 4.

As illustrated in Fig. 7, the intraocular lens 7 which is held by the lens holder 4 is provided so that the rear support part 7c extends rearward from the lens part 7a and the front support part 7b extends forward from the lens part 7a. Note here that in the intraocular lens 7 which is held by the lens holder 4, with reference to a central line X extending along an axis of the plunger 5, a side of the lens part 7a which side is closer to a root of the rear support part 7c is regarded as a Y1 side, and a side of the lens part 7a which side is opposite from the Y1 side is regarded as a Y2 side. According to Embodiment 1, in a case where the injector 1 is placed so that the plunger guide 6 of the injector 1 faces upward and the tip chip 3 faces forward, the Y1 side is the left side, and the Y2 side is the right side.

According to the intraocular lens 7 which is being held by the lens holder 4, a free end 7b1 on an opposite side of a root of the front support part 7b is located on the Y1 side with reference to the central line X, and a free end 7c1 on an opposite side of the root of the rear support part 7c is located on the Y2 side with reference to the central line X.

Furthermore, as illustrated in Fig. 7, the placement surface 41a of the bottom part 41 has, at its rear end, a slope surface 41g. The slope surface 41g projects upward as the slope surface 41g extends rearward from the placement surface 41a which is in front of the slope surface 41g. The slope surface 41g has, at its rear end, an uppermost surface which is located at a position substantially identical to a position at which the side wall part 54b of the L-shaped part 54 of the plunger 5 whose position has been regulated by the plunger position regulating part 41d is located. Thus, at an initial position at which the rear support part 7c is provided so as to be parallel with a bent part of the lens position regulating part 41c, the rear support part 7c of the intraocular lens 7 is provided near the side wall part 54b of the L-shaped part 54 of the plunger 5.

In a case where the plunger 5 which is located at its initial position is pushed by an operator from its initial position to the tip side located in the forward-rearward direction, the L-shaped part 54 of the plunger 5 comes into contact with the rear support part 7c of the intraocular lens 7. This causes the rear support part 7c to be bent toward the lens part 7a. Then, in a case where the plunger 5 further moves to the tip side, the intraocular lens 7 is folded in a recessed shape while passing through the nozzle 32 of the tip chip 3. Subsequently, the intraocular lens 7 reaches the release part 31 while the intraocular lens 7 is being folded. The intraocular lens 7 which has reached the release part 31 is inserted into an eye from a cut side of the release part 31, so that the front support part 7b, the lens part 7a, and the rear support part 7c are inserted in this order.

Note that, in a case where the intraocular lens 7 has passed through the nozzle 32 by an operation to push the plunger 5, the free end 7c1 of the rear support part 7c of the intraocular lens 7 is in contact with a tapered surface of the nozzle 32. In a case where the intraocular lens 7 which is in such a state is pushed into the nozzle 32 and folded, the rear support part 7c which is in contact with the tapered surface of the nozzle 32 may be bent in a rearward unintended direction depending on a location at which the intraocular lens 7 and the L-shaped part 54 of the plunger 5 are in contact with each other. This may cause the intraocular lens 7 to be released while the rear support part 7c is deformed.

In view of the above, the injector 1 in accordance with Embodiment 1 includes a meandering mechanism by which to cause a course of the plunger 5 in the nozzle 32 to meander. The meandering mechanism has, at an entrance of the nozzle 32, the downward protruding part 63 (described earlier) as a contact part which comes into contact with the L-shaped part 54, which is a tip part of the plunger 5. The meandering mechanism has a function of causing the course of the plunger 5 to meander to the Y2 side by a contact between (a) the L-shaped part 54 of the plunger 5 which has been pushed by the operation to push the plunger 5 and (b) the downward protruding part 63.

The inventors of the present invention diligently studied the reason why the rear support part 7c is bent in the rearward unintended direction. As a result, the inventors acquired new knowledge that, in a case where a space between (a) the tapered surface of the nozzle 32 which tapered surface is in contact with the free end 7c1 of the rear support part 7c which is in contact with the plunger 5 and (b) the plunger 5 is made smaller while the intraocular lens 7 is being pushed into the nozzle 32 so as to be folded, the rear support part 7c is folded forward without being bent rearward. The meandering mechanism, which is a characteristic feature of the injector 1 in accordance with Embodiment 1, is based on the new knowledge which has been uniquely acquired by the inventors of the present invention.

The meandering mechanism of the injector 1 in accordance with Embodiment 1 has a function of causing the course of the plunger 5 to meander to the Y2 side by a contact between (a) the L-shaped part 54 of the plunger 5 and (b) the downward protruding part 63. Meandering caused by the meandering mechanism allows a space between (a) a side wall of the nozzle 32 which side wall is provided closer to the free end 7c1 of the rear support part 7c (i.e., on the Y2 side) and (b) the plunger 5. Thus, according to the injector 1 in accordance with Embodiment 1, the intraocular lens 7 can be released from the tip chip 3 while the rear support part 7c is being folded forward without being bent rearward.
(a) of Fig. 8 is a perspective view illustrating a configuration of a meandering mechanism A, which is a characteristic feature of the injector 1 in accordance with Embodiment 1. As illustrated in (a) of Fig. 8, the L-shaped part 54, which is the tip of the plunger 5, has an L-shaped cross section perpendicularly to the shaft of the plunger 5. The bottom part 54a and the side wall part 54b which constitute the L-shaped part 54 are configured to cause the downward protruding part 63 to slide in the bottom part 54a and the side wall part 54b of the plunger 5 which has entered the nozzle 32 through the entrance of the nozzle 32. The meandering mechanism A has not only the downward protruding part 63 (described earlier) but also the projecting part 54c which is provided in the side wall part 54b of the L-shaped part 54 so as to project to the Y1 side.

In a case where the downward protruding part 63 slides in the bottom part 54a and the side wall part 54b of the L-shaped part 54 of the plunger 5, the wall part 64b of the passage part 64 faces the side wall part 54b of the L-shaped part 54 while the downward protruding part 63 is located between the wall part 64b and the side wall part 54b. Meanwhile, the wall part 64a of the passage part 64 faces the side wall part 54b while no downward protruding part 63 is located between the wall part 64a and the side wall part 54b.

According to the configuration, the plunger 5 which is pushed into the entrance of the nozzle 32 by the operation to push the plunger 5 causes the downward protruding part 63 to slide in the bottom part 54a and the side wall part 54b of the L-shaped part 54. The downward protruding part 63 which comes into contact with the projecting part 54c which is provided in the side wall part 54b causes a course of the L-shaped part 54, which is the tip of the plunger 5, to meander to the Y2 side, which is opposite from the Y1 side.

The meandering mechanism A is configured such that the plunger 5 passes through the passage part 64 by coming close to the wall part 64a, which is one of the two wall parts 64a and 64b constituting the passage part 64 and is provided on the Y2 side. This makes it possible to further restrain the rear support part 7c from being bent rearward.

The injector 1 in accordance with Embodiment 1 includes a course changing mechanism B which is provided in the passage part 64 of the plunger guide 6 and by which to reset, to a center of the passage part 64, the course of the plunger 5 which course has been caused, by the meandering mechanism A, to meander. (b) of Fig. 8 illustrates a configuration of the course changing mechanism B and is a view of the plunger guide 6 which is viewed from its lower side and with which the L-shaped part 54 of the plunger 5 is in contact.

As illustrated in (b) of Fig. 8, the wall part 64b which faces the side wall part 54b of the L-shaped part 54 while the downward protruding part 63 is provided between the wall part 64b and the side wall part 54b is linear while extending in the forward-rearward direction. Meanwhile, the wall part 64a which faces the side wall part 54b while no downward protruding part 63 is provided between the wall part 64a and the side wall part 54b has a bent part 65 which is bent so that a distance between a front-side end of the wall part 64a and the wall part 64b is made shorter. That is, the two wall parts 64a and 64b constituting the passage part 64 are provided in parallel with each other on a rear side thereof. On a front side of the two wall parts 64a and 64b, the wall part 64a, which is one of the two wall parts 64a and 64b and is provided on the Y2 side, has the bent part 65 which is bent to the Y1 side.

The bent part 65 functions as the course changing mechanism B. The course of the L-shaped part 54 of the plunger 5 which course has been caused, by a contact between the downward protruding part 63 and the projecting part 54c, to meander to the Y2 side is reset to the center of the passage part 64 by a contact of the L-shaped part 54 with the bent part 65 which is provided on the front side of the wall part 64a. With the configuration, the plunger 5 is caused, by the meandering mechanism A, to meander, and then the course of the plunger 5 is reset to the center of the passage part 64 by the course changing mechanism B, so that the plunger 5 reaches the release part 31 of the tip chip 3. Since the course of the plunger 5 is thus reset to the center before the plunger 5 reaches the release part 31, the intraocular lens 7 can be released while being folded in a balanced recessed shape in the nozzle 32.

According to the injector 1 in accordance with Embodiment 1, the tip chip 3 and the plunger guide 6 are separately formed. Note, however, that the tip chip 3 and the plunger guide 6 can alternatively be integrally formed.

### [Embodiment 2]

The following description will discuss a further embodiment of the present invention. Note that, for convenience, members having functions identical to those of the respective members described in Embodiment 1 are given respective identical reference signs, and a description of those members is omitted here.

An injector in accordance with Embodiment 2 includes a lens holder 4 whose configuration is characteristic. Fig. 9 is a perspective view illustrating a state in which an intraocular lens 7 is placed in a bottom part 41 of the lens holder 4 and a lid part 42 of the lens holder 4 is open. Fig. 10 is a perspective, cross-sectional view illustrating a configuration of the bottom part 41 and the lid part 42 which are provided in the lens holder 4.

The intraocular lens 7 is placed on a placement surface 41a formed in the bottom part 41 of the lens holder 4. While being openably connected to the bottom part 41, the lid part 42 of the lens holder 4 is provided, in a closed state, so as to cover the intraocular lens 7 which has been placed on the placement surface 41a. The lid part 42 includes a groove member 43, an overcover member 44, and a fitted rib member 45.

Fig. 11 is a perspective view illustrating the groove member 43 which is provided in the lid part 42. The groove member 43 has a substantially plate-like shape and is provided, while facing the placement surface 41a across the intraocular lens 7, so as to cover the intraocular lens 7. The groove member 43 is provided with a pair of hollows 49 which extends in the forward-rearward direction and faces the intraocular lens 7 which has been placed in the placement surface 41a.

Fig. 12 is a perspective view illustrating the overcover member 44 which is provided in the lid part 42. The overcover member 44 is formed so as to have a substantially plate-like shape. The overcover member 44 has a curved part 44a which is curved upward while the lid part 42 is closed. The overcover member 44 is provided to the groove member 43 so as to face the placement surface 41a across the groove member 43.

Fig. 13 is a perspective view illustrating the fitted rib member 45 which is provided in the lid part 42. The fitted rib member 45 presses the intraocular lens 7 toward the placement surface 41a by an elastic force by passing through the pair of hollows 49 of the groove member 43 and coming into contact with the overcover member 44 and the intraocular lens 7.

The fitted rib member 45 includes (i) a plate-like base part 46, (ii) a pair of lens pressing parts 47 which protrudes from the base part 46 toward the placement surface 41a through the hollows 49 of the groove member 43, and (iii) an elastically deformed part 48 which protrudes, while curving, from the base part 46 toward the overcover member 44.

The groove member 43, the overcover member 44, and the fitted rib member 45 constitute a "pressing member" of the present invention. Note that the overcover member 44 and the fitted rib member 45 can be integrally formed.

In a case where the intraocular lens 7 is placed on the placement surface 41a of the bottom part 41 of the lens holder 4 thus configured and then the lid part 42 is closed, end surfaces of the lens pressing parts 47 come into contact with the intraocular lens 7, so that the elastically deformed part 48 is pressed against the curved part 44a of the overcover member 44. This causes the elastically deformed part 48 to be elastically deformed by bending. As a result, the fitted rib member 45 is lifted by the intraocular lens 7. The lens pressing parts 47 press the intraocular lens 7 toward the placement surface 41a by an elastic force which is based on elastic deformation, by bending, in the elastically deformed part 48.

In a case where the lid part 42 of the lens holder 4 is thus provided with a movable pressing member configured to press the intraocular lens 7, the intraocular lens 7 is gently pressed against the placement surface 41a of the bottom part 41. Thus, the intraocular lens 7 is placed on the placement surface 41a of the lens holder 4 at all times. This results in avoidance of (i) a problem such that the plunger 5 overrides the intraocular lens 7 and (i) a problem such that the plunger 5 passes under the intraocular lens 7.

Furthermore, it is also possible to prevent occurrence of a problem such that floating of the intraocular lens 7 due to an injected viscoelastic substance which has entered a space below the intraocular lens 7 prevents the intraocular lens 7 from being suitably extruded by the plunger 5.

In a case where the plunger 5 extrudes the intraocular lens 7 by coming into contact with the intraocular lens 7, the lens pressing parts 47 are displaced upward by an elastic force of the elastically deformed part 48. This allows the plunger 5 to suitably extrude the intraocular lens 7.

The intraocular lens 7 is a thin intraocular lens or a thick intraocular lens which has been formed so as to be thicker than the thin intraocular lens. Either the thin intraocular lens or the thick intraocular lens is contained in the lens holder 4. This brings about an effect of stably holding the intraocular lens even in a case where a space is created above the thin intraocular lens while the lens holder 4 is holding the thin intraocular lens.

### [Embodiment 3]

The following description will discuss a further embodiment of the present invention.

Fig. 14 is a perspective view illustrating an overall configuration of an intraocular lens injector 1A in accordance with Embodiment 3. Fig. 15 is an enlarged perspective view illustrating a part of the injector 1A which part is provided with a lens holder 4A. Fig. 16 is an enlarged perspective view illustrating a state in which a lid part 42A which is provided in the lens holder 4A provided to the injector 1A is opened. Fig. 17 is a bottom view of the lid part 42A.

Components similar to those of Embodiments 1 and 2 are given respective similar reference signs. Thus, a specific description of those components is not repeated here.

The injector 1A includes the lens holder 4A which is different in configuration from the lens holder 4 described earlier. The lens holder 4A is constituted by a bottom part 41 and the lid part 42A which is openably connected with the bottom part 41.

Fig. 18 a perspective view illustrating the lens holder 4A which is in a closed state. Fig. 19 a perspective view illustrating the lens holder 4A which is standalone and in which the lid part 42A is in an opened state.

As illustrated in Figs. 16 and 17, the lid part 42A includes a hinge base part 71 (lid body) which has been formed in a plate-like shape. The hinge base part 71 has, at its center, a rectangular opening 76. In the opening 76, a hinge rib member 73 is provided. The hinge rib member 73 has (i) a base part 75 which has been formed in a plate-like shape and is provided so as to be substantially parallel with the hinge base part 71 and (ii) a pair of lens pressing parts 74 which protrudes from the base part 75 toward a placement surface 41a while the lid part 42A is closed. The base part 75 of the hinge rib member 73 is connected to the hinge base part 71 via a pair of hinge parts 72. The base part 75 of the hinge rib member 73 which has been connected to the hinge base part 71 via the pair of hinge parts 72 is elastically deformable by bending while the pair of hinge parts 72 serves as a fulcrum.

The lid part 42A further includes an overcover member 44A which has been formed in a substantially plate-like shape. The overcover member 44A is provided so as to (i) cover the hinge rib member 73 and the hinge base part 71 and (ii) be opposite to the lens pressing parts 74 with respect to the base part 75.

The following description will discuss an operation carried out by the lens holder 4A thus configured. Fig. 20 is a perspective view for describing the operation carried out by the lens holder 4A.

In a case where the lid part 42A of the lens holder 4A is closed, the lens pressing parts 74 of the hinge rib member 73 come into contact with an intraocular lens 7 which has been placed on the placement surface 41a of the bottom part 41. Then, the base part 75 of the hinge rib member 73 which has been connected to the hinge base part 71 via the pair of hinge parts 72 is elastically deformed by bending, in a direction in which the base part 75 is away from the placement surface 41a, while the hinge parts 72 serve as a fulcrum. This causes the lens pressing parts 74 of the hinge rib member 73 to press the intraocular lens 7 toward the placement surface 41a by an elastic force which is based on bending deformation.

Then, in a case where the intraocular lens 7 is pushed, in a forward direction indicated by an arrow A1, by the plunger 5 which has come into contact with the intraocular lens 7 from behind the intraocular lens 7, the base part 75 of the hinge rib member 73 is further elastically formed by bending, in the direction in which the base part 75 is away from the placement surface 41a, while the hinge parts 72 serve as a fulcrum. This causes the lens pressing parts 74 to move from a position, indicated by a solid line L1, to a position, indicated by a broken line L2, so as to be away upward. The lens pressing parts 74 which has been away upward cause the base part 75 to push a central part of the overcover member 44A in an upward direction indicated by an arrow A2, so that the central part of the overcover member 44A is lifted. This makes it possible to prevent the lens pressing parts 74 from preventing movement of the intraocular lens 7 which is pushed by the plunger 5.

The hinge base part 71 and the hinge rib member 73 constitute a "pressing member" of the present invention. The "pressing member" of the present invention can be configured (i) such that a single member such as the fitted rib member 45 described earlier presses the intraocular lens 7 or (ii) such that a member, such as the hinge rib member 73, which is configured to be a part of a lid part by being connected to the hinge base part 71 presses the intraocular lens 7.

The present invention is not limited to the embodiments, but can be altered by a skilled person in the art within the scope of the claims. The present invention also encompasses, in its technical scope, any embodiment derived by combining technical means disclosed in differing embodiments. Further, it is possible to form a new technical feature by combining the technical means disclosed in the respective embodiments.

### [Recap]

An intraocular lens injector in accordance with an embodiment of the present invention includes: a lens holder configured to hold an intraocular lens which has a lens part and a first support part and a second support part which curvedly extend point-symmetrically with respect to the lens part; a plunger configured to extrude the intraocular lens from a rear side of the intraocular lens injector; and a nozzle through which to externally release the intraocular lens, which has been extruded by the plunger, while folding the intraocular lens, the lens holder holding the intraocular lens so that the first support part extends rearward and the second support part extends forward, the intraocular lens injector further including: a meandering mechanism which, assuming that with reference to a central line passing through a center of the lens part and extending in a forward-rearward direction, a side of the lens part which side is closer to a root of the first support part is regarded as a first side and a side of the lens part which side is opposite from the first side is regarded as a second side, has a contact part which is provided at an entrance of the nozzle and comes into contact with a tip of the plunger, the meandering mechanism causing a course of the plunger to meander to the second side by a contact between the contact part and the plunger.

According to the configuration, meandering caused by the meandering mechanism allows a space between (a) a side wall of the nozzle which side wall is provided on the second side of the first support part which extends rearward and (b) the plunger. Thus, according to the configuration, the intraocular lens can be released while the first support part which extends rearward is being folded forward without being bent rearward.

Thus, the configuration makes it possible to achieve an intraocular lens injector which is capable of releasing an intraocular lens while restraining the intraocular lens from being bent.

The intraocular lens injector in accordance with an embodiment of the present invention is preferably configured to further include: a passage part which defines a passage in the nozzle through which passage the tip of the plunger passes, the passage part being provided with a course changing mechanism by which to reset, to a center of the passage part, the course of the plunger which course has been caused, by the meandering mechanism, to meander.

With the configuration, after the plunger has been caused, by the meandering mechanism, to meander, the course of the plunger is reset to the center of the passage part by the course changing mechanism. This allows the intraocular lens to be released while being folded in a balanced recessed shape in the nozzle.

The intraocular lens injector in accordance with an embodiment of the present invention can be configured such that: two side wall parts which constitute the passage part are provided in parallel with each other on a rear side thereof; and on a front side of the two side wall parts, one of the two side wall parts which one is provided on the second side has a bent part which is bent to the first side so as to serve as the course changing mechanism.

According to the configuration, in a case where the tip of the plunger comes into contact with the bent part which is provided on a front side of the side wall part which is provided on the second side, the course is reset to the center of the passage part. This allows the intraocular lens to be released while being folded in a balanced recessed shape in the nozzle.

The intraocular lens injector in accordance with an embodiment of the present invention can be configured such that: the tip of the plunger is an L-shaped part which has an L-shaped cross section perpendicularly to a shaft of the plunger, and a bottom part and a side wall which constitute the L-shaped part are configured to cause the contact part to slide in the bottom part and the side wall of the plunger which has entered the nozzle through the entrance of the nozzle; and the meandering mechanism has a projecting part which is provided on the side wall of the L-shaped part so as to project to the first side.

According to the configuration, the plunger which is pushed into the entrance of the nozzle by an operation to push the plunger causes the contact part to slide in the bottom part and the side wall of the L-shaped part. The contact part which comes into contact with the projecting part which is provided on the side wall causes the course of the plunger to meander to the second side. With the configuration, the intraocular lens can be released while the first support part which extends rearward is being folded forward without being bent rearward.

The intraocular lens injector in accordance with an embodiment of the present invention can be configured to further include: a passage part which defines a passage in the nozzle through which passage the tip of the plunger passes, the meandering mechanism being configured such that the plunger passes through the passage part by coming close to one, provided on the second side, of two side wall parts which constitute the passage part.

With the configuration, the plunger whose course has been caused, by the meandering mechanism, to meander passes through the passage part by coming close to one, provided on the second side, of two side wall parts which constitute the passage part. This makes it possible to further restrain the first support part which extends rearward from being bent rearward.

An intraocular lens injector in accordance with an embodiment of the present invention includes: a body which is tubular; a lens holder provided on an end surface of the body and configured to hold an intraocular lens; a plunger to be inserted into the body, while being capable of entering and leaving the body, so as to (i) come into contact with the intraocular lens which is held by the lens holder and (ii) extrude the intraocular lens; and a nozzle through which to release the intraocular lens, which has been extruded by the plunger, while folding the intraocular lens, the lens holder having: a placement part which is provided with a placement surface on which to place the intraocular lens; and a lid part which is provided so as to cover the placement part, the lid part being provided with a pressing member configured to press the intraocular lens toward the placement surface by an elastic force.

Such a feature allows the intraocular lens which has been placed on the placement surface to be pressed toward the placement surface by an elastic force of a pressing member. Thus, the intraocular lens is stably located with respect to the placement surface. This prevents occurrence of a phenomenon in which a plunger which should extrude the intraocular lens by coming into contact with the intraocular lens overrides the intraocular lens or passes under the intraocular lens. As a result, it is possible to achieve an intraocular lens injector which is capable of suitably extruding an intraocular lens.

The intraocular lens injector in accordance with an embodiment of the present invention is preferably configured such that: the pressing member includes: a groove member which (i) is provided so as to be opposite to the placement surface with respect to the intraocular lens and (ii) is provided with a pair of hollows which faces the intraocular lens; an overcover member which (a) is provided so as to be opposite to the placement surface with respect to the groove member and (b) is provided so as to cover the hollows; and a fitted rib member configured to press the intraocular lens toward the placement surface by an elastic force by passing through the hollows and coming into contact with the overcover member and the intraocular lens which has been placed on the placement surface.

The configuration allows the intraocular lens which has been placed on the placement surface to be pressed toward the placement surface by an elastic force of a pressing member by a simple configuration.

The intraocular lens injector in accordance with an embodiment of the present invention is preferably configured such that the fitted rib member has an elastically deformed part in which elastic deformation by bending occurs due to a contact of the fitted rib member with the overcover member.

The configuration allows an elastic force of a pressing member to be generated by a simple configuration.

An intraocular lens injector in accordance with an embodiment of the present invention includes: a body which is tubular; a lens holder provided on an end surface of the body and configured to hold an intraocular lens; a plunger to be inserted into the body, while being capable of entering and leaving the body, so as to (i) come into contact with the intraocular lens which is held by the lens holder and (ii) extrude the intraocular lens; and a nozzle through which to release the intraocular lens, which has been extruded by the plunger, while folding the intraocular lens, the lens holder having: a placement part which is provided with a placement surface on which to place the intraocular lens; and a lid part which is provided so as to cover the placement part and configured to press the intraocular lens toward the placement surface by an elastic force.

The intraocular lens injector in accordance with an embodiment of the present invention is preferably configured such that: the lid part includes: a groove member which (i) is provided so as to be opposite to the placement surface with respect to the intraocular lens and (ii) is provided with a pair of hollows which faces the intraocular lens; an overcover member which (a) is provided so as to be opposite to the placement surface with respect to the groove member and (b) is provided so as to cover the hollows; and a fitted rib member configured to press the intraocular lens toward the placement surface by an elastic force by passing through the hollows and coming into contact with the overcover member and the intraocular lens which has been placed on the placement surface.

The intraocular lens injector in accordance with an embodiment of the present invention is preferably configured such that: the lid part has: a lid body which has an opening at its center; and a hinge rib member which is (i) provided in the opening, (ii) connected to the lid body via a hinge part so as to be elastically deformable by bending via the hinge part, and (iii) configured to press the intraocular lens toward the placement surface by an elastic force which has been generated by elastic deformation by bending via the hinge part.

### Reference Signs List

- 1: Injector (intraocular lens injector)
- 2: Device body
- 3: Tip chip
- 4: Lens holder
- 5: Plunger
- 6: Plunger guide
- 7: Intraocular lens
- 7a: Lens part
- 7b: Front support part (second support part)
- 7b1, 7c1: Free end
- 7c: Rear support part (first support part)
- 41: Bottom part (placement part)
- 42: Lid part
- 43: Groove member (pressing member)
- 44: Overcover member (pressing member)
- 45: Fitted rib member (pressing member)
- 48: Elastically deformed part (pressing member)
- 54: L-shaped part
- 54a: Bottom part
- 54b: Side wall part
- 54c: Projecting part
- 32: Nozzle
- 63: Downward protruding part (contact part)
- 64: Passage part
- 64a, 64b: Wall parts (two side walls constituting passage part)
- 65: Bent part
- 71: Hinge base part (lid body)
- 72: Hinge part
- 73: Hinge rib member
- 74: Lens pressing parts
- 75: Base part
- 76: Opening
- A: Meandering mechanism
- B: Course changing mechanism

THE FOLLOWING CLAUSES DESCRIBE FURTHER ASPECTS, FEATURES AND EMBODIMENTS OF THE PRESENT DISCLOSURE:
Clause 1 An intraocular lens injector comprising:
   a lens holder configured to hold an intraocular lens which has a lens part and a first support part and a second support part which curvedly extend point-symmetrically with respect to the lens part;
   a plunger configured to extrude the intraocular lens from a rear side of the intraocular lens injector; and
   a nozzle through which to externally release the intraocular lens, which has been extruded by the plunger, while folding the intraocular lens,
   the lens holder holding the intraocular lens so that the first support part extends rearward and the second support part extends forward,
   said intraocular lens injector further comprising:
      a meandering mechanism which, assuming that with reference to a central line passing through a center of the lens part and extending in a forward-rearward direction, a side of the lens part which side is closer to a root of the first support part is regarded as a first side and a side of the lens part which side is opposite from the first side is regarded as a second side, has a contact part which is provided at an entrance of the nozzle and comes into contact with a tip of the plunger, the meandering mechanism causing a course of the plunger to meander to the second side by a contact between the contact part and the plunger.
Clause 2 The intraocular lens injector as set forth in Clause 1, further comprising:
   a passage part which defines a passage in the nozzle through which passage the tip of the plunger passes,
   the passage part being provided with a course changing mechanism by which to reset, to a center of the passage part, the course of the plunger which course has been caused, by the meandering mechanism, to meander.
Clause 3 The intraocular lens injector as set forth in Clause 2, wherein:
   two side wall parts which constitute the passage part are provided in parallel with each other on a rear side thereof; and
   on a front side of the two side wall parts, one of the two side wall parts which one is provided on the second side has a bent part which is bent to the first side so as to serve as the course changing mechanism.
Clause 4 The intraocular lens injector as set forth in any one of Clauses 1 through 3, wherein:
   the tip of the plunger is an L-shaped part which has an L-shaped cross section perpendicularly to a shaft of the plunger, and a bottom part and a side wall which constitute the L-shaped part are configured to cause the contact part to slide in the bottom part and the side wall of the plunger which has entered the nozzle through the entrance of the nozzle; and
   the meandering mechanism has a projecting part which is provided on the side wall of the L-shaped part so as to project to the first side.
Clause 5 The intraocular lens injector as set forth in any one of Clauses 1 through 4, further comprising:
   a passage part which defines a passage in the nozzle through which passage the tip of the plunger passes,
   the meandering mechanism being configured such that the plunger passes through the passage part by coming close to one, provided on the second side, of two side wall parts which constitute the passage part.
Clause 6 An intraocular lens injector comprising:
   a body which is tubular;
   a lens holder provided on an end surface of the body and configured to hold an intraocular lens;
   a plunger to be inserted into the body, while being capable of entering and leaving the body, so as to (i) come into contact with the intraocular lens which is held by the lens holder and (ii) extrude the intraocular lens; and
   a nozzle through which to release the intraocular lens, which has been extruded by the plunger, while folding the intraocular lens,
   the lens holder having:
      a placement part which is provided with a placement surface on which to place the intraocular lens; and
      a lid part which is provided so as to cover the placement part,
   the lid part being provided with a pressing member configured to press the intraocular lens toward the placement surface by an elastic force.
Clause 7 The intraocular lens injector as set forth in Clause 6, wherein:
   the pressing member includes:
   a groove member which (i) is provided so as to be opposite to the placement surface with respect to the intraocular lens and (ii) is provided with a pair of hollows which faces the intraocular lens;
   an overcover member which (a) is provided so as to be opposite to the placement surface with respect to the groove member and (b) is provided so as to cover the hollows; and
   a fitted rib member configured to press the intraocular lens toward the placement surface by an elastic force by passing through the hollows and coming into contact with the overcover member and the intraocular lens which has been placed on the placement surface.
Clause 8 The intraocular lens injector as set forth in Clause 7, wherein the fitted rib member has an elastically deformed part in which elastic deformation by bending occurs due to a contact of the fitted rib member with the overcover member.
Clause 9 An intraocular lens injector comprising:
   a body which is tubular;
   a lens holder provided on an end surface of the body and configured to hold an intraocular lens;
   a plunger to be inserted into the body, while being capable of entering and leaving the body, so as to (i) come into contact with the intraocular lens which is held by the lens holder and (ii) extrude the intraocular lens; and
   a nozzle through which to release the intraocular lens, which has been extruded by the plunger, while folding the intraocular lens,
   the lens holder having:
      a placement part which is provided with a placement surface on which to place the intraocular lens; and
      a lid part which is provided so as to cover the placement part and configured to press the intraocular lens toward the placement surface by an elastic force.
Clause 10 The intraocular lens injector as set forth in Clause 9, wherein:
   the lid part includes:
   a groove member which (i) is provided so as to be opposite to the placement surface with respect to the intraocular lens and (ii) is provided with a pair of hollows which faces the intraocular lens;
   an overcover member which (a) is provided so as to be opposite to the placement surface with respect to the groove member and (b) is provided so as to cover the hollows; and
   a fitted rib member configured to press the intraocular lens toward the placement surface by an elastic force by passing through the hollows and coming into contact with the overcover member and the intraocular lens which has been placed on the placement surface.
Clause 11 The intraocular lens injector as set forth in Clause 9, wherein:
   the lid part has:
   a lid body which has an opening at its center; and
   a hinge rib member which is (i) provided in the opening, (ii) connected to the lid body via a hinge part so as to be elastically deformable by bending via the hinge part, and (iii) configured to press the intraocular lens toward the placement surface by an elastic force which has been generated by elastic deformation by bending via the hinge part.

## Claims

1. An intraocular lens injector comprising:
a lens holder configured to hold an intraocular lens which has a lens part and a first support part and a second support part which curvedly extend point-symmetrically with respect to the lens part;
a plunger configured to extrude the intraocular lens from a rear side of the intraocular lens injector; and
a nozzle through which to externally release the intraocular lens, which has been extruded by the plunger, while folding the intraocular lens,
the lens holder holding the intraocular lens so that the first support part extends rearward and the second support part extends forward,
said intraocular lens injector further comprising:
a meandering mechanism which, assuming that with reference to a central line passing through a center of the lens part and extending in a forward-rearward direction, a side of the lens part which side is closer to a root of the first support part is regarded as a first side and a side of the lens part which side is opposite from the first side is regarded as a second side, has a contact part which is provided at an entrance of the nozzle and comes into contact with a tip of the plunger, the meandering mechanism causing a course of the plunger to meander to the second side by a contact between the contact part and the plunger.

2. The intraocular lens injector as set forth in claim 1, further comprising:
a passage part which defines a passage in the nozzle through which passage the tip of the plunger passes,
the passage part being provided with a course changing mechanism by which to reset, to a center of the passage part, the course of the plunger which course has been caused, by the meandering mechanism, to meander.

3. The intraocular lens injector as set forth in claim 2, wherein:
two side wall parts which constitute the passage part are provided in parallel with each other on a rear side thereof; and
on a front side of the two side wall parts, one of the two side wall parts which one is provided on the second side has a bent part which is bent to the first side so as to serve as the course changing mechanism.

4. The intraocular lens injector as set forth in any one of claims 1 through 3, wherein:
the tip of the plunger is an L-shaped part which has an L-shaped cross section perpendicularly to a shaft of the plunger, and a bottom part and a side wall which constitute the L-shaped part are configured to cause the contact part to slide in the bottom part and the side wall of the plunger which has entered the nozzle through the entrance of the nozzle; and
the meandering mechanism has a projecting part which is provided on the side wall of the L-shaped part so as to project to the first side.

5. The intraocular lens injector as set forth in any one of claims 1 through 4, further comprising:
a passage part which defines a passage in the nozzle through which passage the tip of the plunger passes,
the meandering mechanism being configured such that the plunger passes through the passage part by coming close to one, provided on the second side, of two side wall parts which constitute the passage part.
